# EUROPÄISCHE PATENTSCHRIFT

(11) **EP 2 170 167 B1**
(45) Veröffentlichungstag und Bekanntmachung des Hinweises auf die Patenterteilung: **18.04.2012**
(21) Anmeldenummer: 08786155.5
(22) Anmeldetag: 15.07.2008
(51) Int. Cl.: A61B 5/11

(54) **TRÄGERSTRUKTUR FÜR EIN SENSORBAND**
SUPPORT STRUCTURE FOR A SENSOR STRIP
STRUCTURE DE SUPPORT DESTINÉE À UNE BANDE DE DÉTECTION

(30) Priorität: 18.07.2007 DE 102007034264; 26.09.2007 DE 102007046826
(43) Veröffentlichungstag der Anmeldung: 07.04.2010
(73) Patentinhaber: Siemens Aktiengesellschaft, 80333 München (DE)
(72) Erfinder: BARBOUTIS, Grigorios, 10589 Berlin (DE); GOLDBECK, Dirk David, 81927 München (DE); HAPPEL, Tobias, 10553 Berlin (DE); KWIATEK, Andre Matthias, 12159 Berlin (DE); NERRETER, Stefan, 15754 Heidesee Ot Blossin (DE)
(86) Internationale Anmeldenummer: PCT/EP2008/059253
(87) Internationale Veröffentlichungsnummer: WO 2009/010518

(56) Entgegenhaltungen:
- WO-A-01/58538
- WO-A-97/43953
- GB-A- 2 377 157
- US-A- 5 316 017
- US-A- 6 032 530

## Beschreibung

Die Erfindung betrifft eine biegeschlaffe Trägerstruktur für ein Sensorband zur konturnahen Befestigung an einer Oberfläche, insbesondere derjenigen eines menschlichen oder tierischen Körpers. Außerdem ist vorgesehen, dass die Trägerstruktur einen Kanal für das Sensorband aufweist, in dem das Sensorband längs verschiebbar gelagert werden kann. Durch die längs verschiebliche Lagerung kann sich vorteilhaft die Trägerstruktur unabhängig von dem Sensorband dehnen bzw. stauchen, wenn die Oberfläche, beispielsweise der Rücken eines menschlichen Körpers, Bewegungen ausführt. Hierbei gleitet das Sensorband widerstandsarm in dem Kanal, wobei der Kanal hierbei eine konturnahe Führung des Sensorbandes bewirkt, so dass die Biegung der Oberfläche mit hoher Genauigkeit gemessen werden kann.

Eine biegeschlaffe Trägerstruktur der eingangs genannten Art ist beispielsweise aus der US 7,033,281 B2 bekannt. Die Trägerstruktur besteht aus einer Manschette, die ein Sensorband zur Ermittlung des Knickwinkels des Ellenbogens eines menschlichen Armes trägt. Zu Messzwecken wird die Manschette auf den Arm aufgeschoben, wobei diese den Arm vollumfänglich umschließt und derart positioniert wird, dass der Ellenbogen sich in der Manschette befindet. Wird der Knickwinkel des Ellenbogengelenkes verändert, so kann dies durch die Knickung des Sensorbandes nachgewiesen werden. Hierbei wird hingenommen, dass die Manschette eine gewisse Einschränkung der Bewegungsfreiheit des Armes bewirkt.

Um eine möglichst genaue Messung der Rückenbiegung durchführen zu können, ist weiterhin vorgesehen, dass die Trägerstruktur eine im Wesentlichen punktförmige Fixierungsvorrichtung aufweist, mit der das Sensorband an einem Referenzpunkt unverschiebbar auf dem Trägerband gehalten werden kann. Mit einer im Wesentlichen punktförmigen Fixierungsvorrichtung ist gemeint, dass das Sensorband lediglich an einer Stelle, vorzugsweise einem Ende gehalten ist, so dass der restliche Teil des Sensorbandes in dem Kanal verschiebbar ist. Dies wird notwendig, da das Sensorband hinsichtlich einer Längendehnung wesentlich steifer ausgeführt ist, als die elastische Trägerstruktur. Andererseits muss eine zuverlässige Positionierung der Sensorstruktur auf den Rücken oder einer anderen Oberfläche gewährleistet sein, damit das Sensorband nicht unter Erzeugung von Messfehlern undefiniert in dem Kanal hin- und herrutschen kann.

Gemäß der US 6,032,530, der WO 01/58538 A1 und der WO 97/43953 A1 ist es bekannt, dass Sensorbänder zur Ermittlung der Biegung von Körperteilen, beispielsweise der Hand, in einem Handschuh oder einer Gelenkmanschette untergebracht werden können. Der Proband kann diesen Handschuh oder die Manschette dann anziehen bzw. anlegen, wobei die Sensorbänder bei Bewegungen dieses Kleidungsstückes gebogen werden und entsprechende Sensorsignale erzeugt werden. Gemäß der WO 97/43953 A1 ist es überdies bekannt, dass die verwendeten Sensorbänder mittels einer aufgenähten Tasche an dem Handschuh fixiert werden können.

Die Aufgabe der Erfindung besteht darin, eine Trägerstruktur für ein Sensorband anzugeben, welches nach der konturnahen Befestigung auf der Oberfläche eine Bewegung des die Oberfläche bildenden Körpers möglichst wenig beeinflusst.

Diese Aufgabe wird mit der in Anspruch 1 definierten Trägerstruktur erfindungsgemäß dadurch gelöst, dass die Trägerstruktur ein Trägerband aufweist, welches mit der einen Seite auf der Oberfläche befestigt werden kann und dessen andere Seite einen Wandteil des Kanals bildet. Hierdurch wird eine Trägerstruktur erhalten, welche vorteilhaft an die Geometrie des Bandes weitgehend angepasst ist. Hierdurch lässt sich diese vorteilhaft vergleichsweise komfortabel auf der Oberfläche, insbesondere der Haut eines Probanden, anbringen. Anders als die Manschette gemäß dem Stand der Technik schnürt die Trägerstruktur das zu messende Objekt nicht ein, wodurch die Bewegungsfreiheit weitgehend erhalten bleibt. Auch wird der zur elastischen Verformung der Trägerstruktur erforderliche Kraftaufwand aufgrund des minimalen Materialeinsatzes vorteilhaft verringert, weswegen das Tragen des Trägerbandes durch den Probanden als weniger störend empfunden wird.

Außerdem ist erfindungsgemäß vorgesehen, dass das Trägerband unabhängig von seiner Längsdehnung seine Breite zumindest im Wesentlichen beibehält. Dies kann beispielsweise durch Gewebebänder erreicht werden, deren Breite sich bei einer Längung bzw. Verkürzung nicht verändert. Derartige Bänder werden beispielsweise unter dem Handelsnamen CureTape ® vertrieben. Durch ein in der Breite konstantes Band als Trägerstruktur werden vorrangig folgende Vorteile erreicht. Zum einen wird die Haut (allgemein Oberfläche) bei einer Dehnung, wie sie beispielsweise am Rücken beobachtet wird, nicht durch das sich in Querrichtung zusammenziehende Band zu unnatürlichen Verformungen gezwungen, denn die Haut vollführt beispielsweise bei einer Flexion des Rückens ebenfalls nur elastische Verformungen in Biegerichtungen. Ein weiterer Vorteil besteht darin, dass der auf der Trägerstruktur ausgebildete Kanal bei einer Verbiegung der Trägerstruktur im Wesentlichen die gleiche Breite behält, so dass unabhängig von der Biegelinie des Trägerbandes (zumindest in einem definierten Betriebsbereich) ein kräftearmes Gleiten des Sensorbandes im Kanal gewährleistet bleibt.Hierzu ist erfindungsgemäß vorgesehen, dass die Trägerstruktur in Richtung des Verlaufes des Kanals elastisch ist. Der Vorteil kann insbesondere bei der bereits erwähnten Verwendung der Trägerstruktur auf einem Rücken einfach erklärt werden. Wenn die Wirbelsäule unterschiedliche Krümmungen (beispielsweise Flexion und Extension, d. h. Biegungen nach vorn und zurück) durchführt, wird die Rückenhaut gedehnt bzw. zieht sich zusammen. Um einen vorteilhaft großen Tragekomfort verbunden mit einer möglichst geringen Störung des natürlichen Bewegungsablaufes zu gewährleisten, kann die elastische Trägerstruktur diese Bewegung nachvollziehen, indem diese auf dem Rücken fixiert wird. Hierzu ist beispielsweise ein hautverträglicher Kleber geeignet, der die Trägerstruktur der Länge nach fest mit dem Rücken verbindet.

Gemäß einer Ausgestaltung der Erfindung ist vorgesehen, dass der Kanal im unverformten Zustand der Trägerstruktur länger ist, als der in ihm zu führende Teil des Sensorbandes. Hierdurch wird vorteilhaft erreicht, dass sowohl bei einer Verkürzung als auch bei einer Verlängerung der Trägerstruktur eine Führung des Sensorbandes in dem Kanal möglich ist. Im Fall der Verwendung eines Rückensensors kann somit sowohl eine Flexion als auch eine Extension ausgeführt werden, wenn die Trägerstruktur bei gerade ausgerichtetem Rücken auf diesem platziert wird.

Weiterhin ist erfindungsgemäß vorgeschen, dass der Kanal durch einen elastischen Textilstreifen gebildet ist, der zumindest entlang seiner Seitenränder unter Ausbildung einer Tasche fest mit der restlichen Trägerstruktur verbunden ist. Der elastische Textilstreifen schmiegt sich vorteilhaft an die Unterlage an, auf der er befestigt ist. Diese Unterlage wird durch den größeren Teil der Trägerstruktur gebildet. Soll nun ein Sensorband auf der Trägerstruktur befestigt werden, so kann dieser vorteilhaft auf einfache Weise in die gebildete Tasche eingeschoben werden. Dabei gibt der Textilstreifen nach, so dass das Sensorband auch mit einer Verdickung, beispielsweise einer am Ende befindlichen Auswertungseinheit, in den gebildeten Kanal eingeschoben werden kann. Dabei wird aufgrund der Rückstellkräfte des elastischen Textilstreifens ein gewisser Anpressdruck auf das Sensorband ausgeübt, so dass dieses zu - verlässig auf der Trägerstruktur anliegt. Gleichzeitig bleibt aber die Verschiebbarkeit in Längsrichtung des taschenförmigen Kanals erhalten.

Besonders vorteilhaft ist die Kombination einer Trägerstruktur in Form eines Trägerbandes mit dem elastischen Textilstreifen. Das Trägerband muss hierbei lediglich so breit ausgeführt werden, dass beidseitig des Kanals eine zuverlässige Verbindung der Trägerstruktur mit dem Textilstreifen sichergestellt werden kann. Hierbei ist es von besonderem Vorteil, wenn der Textilstreifen eine geringere Dehnsteifigkeit als die restliche Trägerstruktur, insbesondere das Trägerband, aufweist. Hierdurch wird vorteilhaft eine Aufgabenteilung erreicht, wie sie bereits im Zusammenhang mit dem Einsatz der Trägerstruktur und dem flexiblen Textilstreifen oben angegeben wurde. Der Textilstreifen drückt das Sensorband zuverlässig gegen die Trägerstruktur und ermöglicht hierbei gleichzeitig eine weitgehend kräftefreie Führung des Sensorbandes im Kanal. Die Trägerstruktur (insbesondere bandförmig) ermöglicht eine zuverlässige Befestigung auf der Oberfläche (Rücken) und ermöglicht hierbei gleichzeitig eine Längenänderung dieser Oberfläche.

Weiterhin ist es vorteilhaft, wenn der Kanal durch voneinander beabstandete Schutzbügel gebildet ist, welche in einer Ausrichtung längs zum Kanal auf der Trägerstruktur fixiert sind und den Einbauort des Sensorbandes überspannen. Derartige Schutzbügel bilden somit brückenartige Führungselemente für das Sensorband aus und bilden hierdurch den zur Führung vorgesehenen Kanal. Außerdem ermöglicht die beabstandete Fixierung auf dem Trägerband die bereits angesprochene Längenänderung des Trägerbandes (bzw. der Trägerstruktur), da bei einer elastischen Verformung des Trägerbandes der Abstand der einzelnen Schutzbügel zueinander veränderlich ist.

Die Schutzbügel haben den wesentlichen Vorteil, dass das Sensorband vor Beschädigungen geschützt werden kann. Im Fall der Anwendung als Rückensensor ist es beispielsweise möglich, dass sich der Proband an einer Tischkante anlehnt, die örtlich quer zur Längenausdehnung des Sensorbandes in dieses einschneidet. Dies kann zu stark verfälschten Messergebnissen bzw. einer Beschädigung (beispielsweise Bruch) des Sensorbandes führen. Die Schutzbügel sind genügend stabil ausgeführt, um derartige Krafteinwirkungen auf das Sensorband aufzufangen.

Selbstverständlich kann die Verwendung von Schutzbügeln auch mit einem elastischen Textilstreifen zur Ausbildung des Kanals kombiniert werden. Hierbei wird vorteilhaft eine Aufgabenteilung erreicht, wobei die Führung des Sensorbandes vorrangig durch den taschenförmigen Kanal des Textilstreifens übernommen wird. Die Schutzbügel überspannen diese Tasche dann mit einem bestimmten Abstand zum Sensorband, so dass eine Berührung unter normalen Betriebsbedingungen nicht zustande kommt. Dies hat den Vorteil, dass eine gewisse elastische Verformung der Schutzbügel hingenommen werden kann, wenn diese auf die Trägerstruktur eingeprägte Kräfte auffangen. Hierdurch können die Schutzbügel weniger massiv ausgelegt werden, was vorteilhaft den Tragekomfort der Trägerstruktur verbessert.

Zusätzlich ist es vorteilhaft, wenn der Kanal und/oder das Sensorband mit einer die Reibung vermindernden Beschichtung versehen ist. Diese Beschichtung kann fest sein, beispielsweise kann auf das Sensorband eine Teflonbeschichtung aufgebracht werden. Eine andere Möglichkeit ist unter der Voraussetzung einer dichten Ausführung des Kanals die Verwendung von Schmierstoffen wie Glyzerin oder einem Gleitgel.

Zur Erläuterung wird im Weiteren ein Sensorband zur Montage in einer biegeschlaffen Trägerstruktur beschrieben, welche eine konturnahe Befestigung an einer Oberfläche, insbesondere derjenigen eines menschlichen oder tierischen Körpers ermöglicht. Ein derartiges Sensorband ist in dem eingangs bereits angegebenen Stand der Technik beschrieben.

Das Sensorband weist einen Referenzpunkt auf, der mit einer im Wesentlichen punktförmigen Fixierungsvorrichtung der Trägerstruktur verbunden werden kann, wobei der Referenzpunkt unverschiebbar auf der Trägerstruktur gehalten wird. Durch die Ausbildung des Sensorbandes mit einem Referenzpunkt werden die bereits beschriebenen Vorteile erreicht, nämlich dass einerseits die Position des Sensorbandes auf der Trägerstruktur zumindest in einem Punkt klar definiert ist und andererseits eine longitudinale Verschiebbarkeit des Sensorbandes auf der Tragstruktur möglich ist, wenn die Trägerstruktur eine elastische Längenänderung erfährt.

Weitere Einzelheiten der Erfindung werden im Folgenden anhand der Zeichnung beschrieben. Gleiche oder sich entsprechende Zeichnungselemente sind in den einzelnen Figuren jeweils mit den gleichen Bezugszeichen versehen und werden nur insoweit mehrfach erläutert, wie sich Unterschiede zwischen den einzelnen Figuren ergeben. Es zeigen
- Figur 1: ein Ausführungsbeispiel der erfindungsgemäßen Trägerstruktur als Aufsicht,
- Figur 2: die erfindungsgemäße Trägerstruktur gemäß Figur 1 ohne den in Figur 1 dargestellten Textilstreifen und mit einem eingebauten Ausführungsbeispiel eines Sensorbandes und
- Figur 3: die Schnitte III.1 und III.2 gemäß Figur 2.

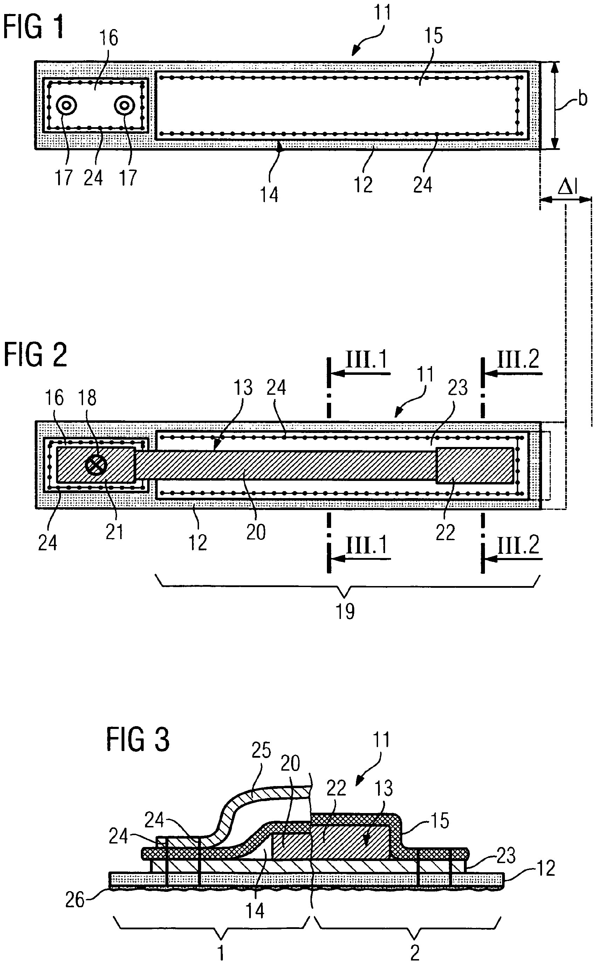

Eine Trägerstruktur 11 gemäß Figur 1 besteht aus einem Trägerband 12, welches die Grundlage der Trägerstruktur bildet. Die Trägerstruktur dient zur Aufnahme eines Sensorbandes 13 (vgl. Figur 2), welches zur zuverlässigen Lagerung in eine Tasche 14 eingeschoben werden kann, welche durch einen auf das Trägerband 12 aufgenähten Textilstreifen 15 gebildet ist. Die Tasche 14 ist auf der einen Seite geschlossen und auf der anderen Seite offen, so dass von der offenen Seite aus das Sensorband 13 eingeschoben werden kann. Vor der offenen Seite der Tasche 14 ist weiterhin ein verhältnismäßig steifer Flicken 16 aufgenäht, der zwei Druckknopfverschlüsse 17 als Fixierungsvorrichtung für das Sensorband 13 trägt. Durch die Steifigkeit des Flickens ist gewährleistet, dass dieser Bereich mit den Druckknopfverschlüssen 17 die in Figur 1 angedeuteten Längenänderungen Δl des Trägerbandes nicht nachvollzieht, so dass dieser Bereich als Referenzpunkt 18 des Sensorbandes 13 verwendet werden kann. Das Sensorband 13 hat zur Fixierung auf dem Trägerband 12 korrespondierende Druckknopfverschlüsse, die in Figur 2 nicht zu erkennen sind, da sie der Bildfläche abgekehrt sind.

Die Figur 2 beinhaltet die Darstellung des auf dem Trägerband 12 montierten Sensorbandes 13. Das Sensorband besteht genau genommen aus dem eigentlichen sensitiven Bereich 20, einer Sendeeinheit 21 und einer Empfangseinheit 22. Eine Kontaktierung ist der Einfachheit halber weggelassen oder könnte beispielsweise kabellos mittels einer Funkverbindung hergestellt werden. Der Textilstreifen 15 ist in Figur 2 nicht dargestellt, wenn auch vorhanden. Es wird deutlich, dass auf dem Trägerband 12 eine Beschichtung 23 vorgesehen ist, die die Gleiteigenschaften verbessert und deren Ausdehnung derjenigen des Textilstreifens 15 entspricht. Den Figuren 1 und 2 ist weiterhin zu entnehmen, dass eine Verbindung der einzelnen Bauteile der Trägerstruktur 11 beispielsweise durch Nähte 24 erfolgen kann. Eine andere Möglichkeit besteht darin, die Bauteile miteinander zu verschweißen oder zu verkleben. Es ist zu beachten, dass die Verbindungstechnik die Längenänderung des Trägerbandes 12 mitvollziehen muss. Ein Verschweißen würde beispielsweise durch einzelne Schweißpunkte möglich sein. Klebeverbindungen müssten in ihren Elastizitätswerten angepasst sein. Als Naht muss eine dehnbare Naht, beispielsweise eine Zick-Zack-Naht verwendet werden.

Der Figur 2 ist weiterhin zu entnehmen, dass das Sensorband in dem verkürzten Zustand dargestellt ist, bei dem die verkürzte Länge der Tasche 14 gerade noch zur Aufnahme des Sensorbandes 13 ausreicht. Strichpunktier dargestellt ist die Länge des Trägerbandes 12 incl. Tasche 14 im entspannten Zustand. Angedeutet ist weiterhin strichpunktiert, dass das Trägerband auch um die Hälfte von Δl gelängt werden kann.

In der Figur 3 ist die Trägerstruktur 11 mit dem Sensorband 13 im Querschnitt zu erkennen. Hierbei sind die Schnittebenen III.1 und III.2 gemäß Figur 2 dargestellt und mit 1 und 2 gekennzeichnet. Es wird deutlich, dass sich der flexible Textilstreifen 15 jeweils an die Kontur des Sensorbandes 13 anpassen kann, wodurch gewährleistet ist, dass dieses immer auf die Unterlage des mit der Beschichtung 23 versehenen Trägerbandes 12 angepresst wird. Weiterhin ist im Schnitt III.1 ein Schutzbügel 25 zu erkennen, der ebenfalls auf dem Trägerband festgenäht ist. Die Nähte 24 sind ebenfalls angedeutet. Zuletzt ist zu erkennen, dass das Trägerband 12 auf der Unterseite, die auf der Oberfläche befestigt werden soll, eine Klebeschicht 26 aufweist.

Folgende Materialien können für die Trägerstruktur zum Einsatz kommen. Das Trägerband 12 kann aus dem unter dem Handelsnamen CureTape ® vertriebenen Band bestehen. Dieses ist entlang seiner Längsausdehnung elastisch (Δl), schnürt sich jedoch bei Längung nicht ein, sondern behält seine Breite b bei. Dieses Band ist an der Unterseite mit einer hautverträglichen Klebeschicht 26 beschichtet und kann so fest auf der Haut eines Probanten beispielsweise auf dem Rücken befestigt werden. Bei der Beschichtung 23 kann es sich beispielsweise um eine Teflonbeschichtung handeln, die auf das Trägerband 12 aufgeschleudert wird. Diese ist wesentlich dünner ausgebildet, als in Figur 3 dargestellt, so dass sie die notwendige Elastizität aufbringt, wenn das Trägerband um den Betrag Δl in seiner Länge verändert wird. Der Textilstreifen 15 kann aus einem hochelastischen Stoff, wie er beispielsweise zur Herstellung von Badeanzügen entwickelt wurde, bestehen, beispielsweise Lycra ® oder Elastan ®. Der Bügel 25 kann aus einem thermoplastischen Kunststoff bestehen. Dieser weist einerseits eine genügende Bruchsicherheit und Eigenstabilität auf, um einen Schutz des Sensorbandes 20 zu gewährleisten. Andererseits ist er flexibel genug, damit er den Verformungen des Trägerbandes 12 folgen kann. Wird der Trägerbügel mit geeigneten Löchern versehen, lässt sich eine Befestigung mittels der Naht 24 erreichen.

Das Sensorband ist biegesensitiv ausgestaltet. Auf diese Weise lassen sich die in die Trägerstruktur eingeprägten Biegungen bestimmen. Dabei können unterschiedliche Wirkprinzipien für das Sensorband umgesetzt werden. Bevorzugt kann das Sensorband 13 wie dargestellt ein optisches Sensorband sein, wobei dieses optische Wellenleiter enthält, durch die ein Messsignal gesendet wird. Abhängig von der Biegung der Sensorfasern wird unterschiedlich viel Licht aus den Sensorfasern ausgekoppelt, so dass durch Bestimmung des durchgeleiteten Lichtes in der Empfängereinheit 22 ein Rückschluss auf den vorliegenden Biegungszustand der Sensorfaser und damit der Trägerstruktur 11 möglich ist. Das Licht wird über die Sendeeinheit 21 in den sensitiven Bereich 20, gebildet durch die Lichtleitfasern (nicht dargestellt), eingeleitet.

Selbstverständlich sind auch andere Messprinzipien denkbar, beispielsweise ist eine elektrische Auslesung von Messsignalen möglich (kapazitive oder resistive Elemente).

## Patentansprüche

1. Biegeschlaffe Trägerstruktur für ein Sensorband (13) zur konturnahen Befestigung an einer Oberfläche, insbesondere derjenigen eines menschlichen oder tierischen Körpers, wobei die Trägerstruktur
- einen Kanal (19) für das Sensorband (13) aufweist, in dem das Sensorband (13) längs verschiebbar gelagert werden kann,
- in Richtung des Verlaufes des Kanals (19) elastisch ist und
- eine im Wesentlichen punktförmige Fixierungsvorrichtung (16,17) aufweist, mit der das Sensorband an einem Referenzpunkt (18) unverschiebbar auf der Trägerstruktur gehalten werden kann,
**dadurch gekennzeichnet, dass**
die Trägerstruktur durch ein Trägerband (12) und einen Textilstreifen (15) gebildet ist, wobei
- das Trägerband (12) auf der einen Seite eine Klebeschicht (26) zur Befestigung auf der Oberfläche aufweist und seine andere Seite einen Wandteil des Kanals bildet,
- der andere Wandteil des Kanals durch den elastischen Textilstreifen (15) gebildet ist, der zumindest entlang seiner Seitenränder unter Ausbildung einer Tasche (15) fest mit dem Trägerband (12) verbunden ist und
- das Trägerband (12) unabhängig von seiner Längsdehnung seine Breite zumindest im Wesentlichen beibehält.

2. Trägerstruktur nach Anspruch 1,
**dadurch gekennzeichnet,**
**dass** der Kanal (19) im unverformten Zustand der Trägerstruktur länger ist, als der in ihm zu führende Teil des Sensorbandes (13).

3. Trägerstruktur nach Anspruch 1 oder 2,
**dadurch gekennzeichnet,**
**dass** der Textilstreifen (15) eine geringere Dehnsteifigkeit als die restliche Trägerstruktur, insbesondere das Trägerband (12), aufweist.

4. Trägerstruktur nach einem der voranstehenden Ansprüche,
**dadurch gekennzeichnet,**
**dass** der Kanal durch voneinander beabstandete Schutzbügel (25) mitgebildet ist, welche in einer Ausrichtung längs zum Kanal (19) auf der Trägerstruktur fixiert sind und den Einbauort des Sensorbandes überspannen.

5. Trägerband nach einem der voranstehenden Ansprüche,
**dadurch gekennzeichnet,**
**dass** der Kanal (19) mit einer die Reibung vermindernden Beschichtung versehen ist.

## Claims

1. Flexurally limp carrier structure for a sensor tape (13) for near-contour fastening on a surface, in particular that of a human or animal body, wherein the carrier structure
- has for the sensor tape (13) a channel (19) in which the sensor tape (13) can be mounted so as to be longitudinally displaceable,
- is elastic in the direction of run of the channel (19) and
- has an essentially punctiform fixing device (16, 17), by means of which the sensor tape can be held non-displaceably on the carrier structure at a reference point (18),
**characterized in that** the carrier structure is formed by a carrier tape (12) and a textile strip (15), wherein
- the carrier tape (12) has an adhesive layer (26) on one side for fastening to the surface and its other side forms a wall part of the channel,
- the other wall part of the channel is formed by the elastic textile strip (15) which is firmly connected, at least along its side margins, to the carrier tape (12) so as to form a pocket (15) and
- the carrier tape (12) at least essentially preserves its width independently of its elongation.

2. Carrier structure according to Claim 1, **characterized in that**, in the non-deformed state of the carrier structure, the channel (19) is longer than that part of the sensor tape (13) which is to be guided in it.

3. Carrier structure according to Claims 1 or 2, **characterized in that** the textile strip (15) has a lower rigidity under extension than the remaining carrier structure, in particular the carrier tape (12).

4. Carrier structure as claimed in one of the preceding claims, **characterized in that** the channel is also formed by guard bridges (25) which are spaced apart from one another and which are fixed on the carrier structure in a longitudinal orientation with respect to the channel (19), and span the place of installation of the sensor tape.

5. Carrier tape as claimed in one of the preceding claims, **characterized in that** the channel (19) is provided with a coating reducing the friction.

## Revendications

1. Structure de support à flexion molle, pour un ruban ( 13 ) formant capteur, en vue de la fixation proche du contour sur une surface, notamment celle d'un corps humain ou animal, dans laquelle la structure de support
- a un canal ( 19 ) pour le ruban ( 13 ) formant capteur, canal dans lequel le ruban ( 13 ) formant capteur peut être monté coulissant dans la longueur,
- est élastique dans la direction dans laquelle s'étend le canal ( 19 ) et
- a un dispositif ( 16, 17 ) d'immobilisation, essentiellement ponctuel, par lequel le ruban formant capteur peut être maintenu en un point ( 18 ) de référence, sans possibilité de se déplacer sur la structure de support,
**caractérisée en ce que** la structure de support est formée par un ruban ( 12 ) de support et par une bande ( 15 ) textile, dans laquelle
- la bande ( 12 ) de support a, d'un côté, une couche ( 26 ) de colle pour la fixation sur la surface et, de l'autre côté, forme une partie de paroi du canal,
- l'autre partie de paroi du canal est formée par la bande ( 15 ) textile élastique, qui est reliée rigidement, au moins le long de ses bords latéraux, au ruban ( 12 ) de support avec formation d'une poche ( 15 ) et
- le ruban ( 12 ) de support conserve, au moins sensiblement, sa largeur indépendamment de son allongement en longueur.

2. Structure de support suivant la revendication 1,
**caractérisée**
**en ce que** le canal ( 19 ) est, à l'état non déformé de la structure de support, plus long que la partie y passant du ruban ( 13 ) formant capteur.

3. Structure de support suivant la revendication 1 ou 2,
**caractérisée**
**en ce que** la bande ( 15 ) textile a une aptitude à s'allonger plus petite que le reste de la structure de support, notamment que le ruban ( 12 ) de support.

4. Structure de support suivant l'une des revendications précédentes,
**caractérisée**
**en ce que** le canal est formé par des étriers ( 25 ) de protection, qui sont immobilisés sur la structure de support dans une direction le long du canal ( 19 ) et qui recouvrent l'emplacement où est mis le ruban formant capteur.

5. Structure de support suivant l'une des revendications précédentes,
**caractérisée**
**en ce que** le canal ( 19 ) est pourvu d'un revêtement diminuant le frottement.
